(19) Europäisches Patentamt
European Patent Office
Office européen des brevets

(11) Veröffentlichungsnummer: **0 271 817 B1**

(12) **EUROPÄISCHE PATENTSCHRIFT**

(45) Veröffentlichungstag der Patentschrift: **25.11.92**

(51) Int. Cl.⁵: **C07D 201/08**

(21) Anmeldenummer: **87118201.0**

(22) Anmeldetag: **09.12.87**

# (54) Verfahren zur Herstellung von Caprolactam aus 6-Aminocapronsäure, deren Estern und Amiden.

(30) Priorität: **17.12.86 DE 3643011**

(43) Veröffentlichungstag der Anmeldung:
**22.06.88 Patentblatt 88/25**

(45) Bekanntmachung des Hinweises auf die Patenterteilung:
**25.11.92 Patentblatt 92/48**

(84) Benannte Vertragsstaaten:
**BE CH DE ES FR GB IT LI NL**

(56) Entgegenhaltungen:
**CH-A- 334 311**
**DE-A- 2 535 689**
**DE-A- 3 235 938**
**DE-C- 859 015**
**GB-A- 1 268 869**

**Chemical Abstracts, Band 81, Nr.21, 25. November 1974, Columbus, Ohio, USA, T.V.Stezhko et al., Seite 405, Spalte 1; & Zh. Org. Khim. 1974, 10(7), 1556**

(73) Patentinhaber: **BASF Aktiengesellschaft**
**Carl-Bosch-Strasse 38**
**W-6700 Ludwigshafen(DE)**

(72) Erfinder: **Dockner, Toni, Dr.**
**Grossgasse 6**
**W-6701 Meckenheim(DE)**
Erfinder: **Sauerwald, Manfred**
**Gebhardtstrasse 16**
**W-6701 Roedersheim-Gronau(DE)**
Erfinder: **Fischer, Rolf, Dr.**
**Bergstrasse 98**
**W-6900 Heidelberg(DE)**
Erfinder: **Hutmacher, Hans-Martin, Dr.**
**Ruedigerstrasse 70**
**W-6700 Ludwigshafen(DE)**
Erfinder: **Priester, Claus-Ulrich, Dr.**
**Wiesenstrasse 18**
**W-6701 Meckenheim(DE)**
Erfinder: **Vagt, Uwe, Dr.**
**Paul-Neumann-Strasse 44**
**W-6720 Speyer(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Gegenstand der Erfindung ist ein Verfahren zur Herstellung von Caprolactam aus 6-Aminocapronsäure, deren Estern und Amiden sowie Gemischen derselben.

In der DE-OS 22 49 993 wird ein Verfahren beschrieben, bei dem man 6-Aminocapronsäureester in Gegenwart von Wasser bei einer Temperatur von 250 bis 350° C zu Caprolactam umsetzt. Das Verfahren hat jedoch den Nachteil, daß die Umsetzung unter erhöhtem Druck durchgeführt werden muß. Zudem soll die Cyclisierung bei niederen Konzentrationen durchgeführt werden, um die Bildung von Oligomeren zu unterdrücken. Dies hat zur Folge, daß sich die Isolierung des Caprolactams aus der verdünnten Lösung technisch aufwendig gestaltet. Nach einem anderen in der DE-OS 32 35 938 beschriebenen Verfahren werden 6-Aminocapronsäureester durch Erhitzten auf 180 bis 250° C in einem mehrwertigen Alkohol mit einem Siedepunkt, der höher als derjenige des Caprolactams ist, in einem Zeitraum von 0,5 bis 5 Stunden zu Caprolactam umgesetzt und anschließend Caprolactam aus dem erhaltenen Reaktionsgemisch durch Destillation abgetrennt. Dieses Verfahren hat den Nachteil, daß ebenfalls relativ verdünnte Lösungen mit 5 bis 15 Gew.-% angewandt werden sollen und zudem anspruchsvolle Lösungsmittel wie Tetraethylenglykol, die zurückgewonnen werden müssen, angewandt werden. Schließlich gestaltet sich die Gewinnung des Caprolactams durch zwei getrennte Verfahrensstufen aufwendig.

In der GB-A 12 68 869 wird ein Verfahren zur Herstellung von Caprolactam beschrieben, bei dem man 6-Aminocapronsäure oder dessen Amid unter Durchleiten von Wasserdampf auf eine Temperatur von 150 bis 400° C erhitzt und Caprolactam mit dem Nasserdampf entfernt.

Ferner wird in der DE-A 25 35 689 beschrieben, daß Caprolactam hergestellt wird durch Erhitzen von 6-Aminocapronsäure gelöst in einem Lösungsmittel, das wenigstens 60 Vol.-% Ethanol oder Methanol enthält, auf 170 bis 200° C.

In Ind. Eng. Chem. Process Des. Dev., Vol. 17 (1978), Seite 9, wird auch schon die Cyclisierung von 6-Aminocapronsäureamid in Ethanol und Wasser bei einer Temperatur von 180 bis 250° C zu Caprolactam beschrieben. Hierbei werden jedoch erhebliche Mengen an Dimeren als Nebenprodukte gebildet, wodurch die Ausbeute zu wünschen übrig läßt.

Es war deshalb die technische Aufgabe gestellt, ein Verfahren zur Herstellung von Caprolactam, ausgehend von 6-Aminocapronsäure, deren Estern oder Amiden oder Gemischen derselben zur Verfügung zu stellen, das drucklos mit hohen Ausbeuten in kurzer Zeit verläuft und keine aufwendigen Lösungsmittel erfordert und zudem eine einfache Abtrennung des Caprolactams ermöglicht.

Diese technische Aufgabe wird gelöst in einem Verfahren zur Herstellung von Caprolactam durch Erhitzen von 6-Aminocapronsäure, deren Estern oder Amiden der allgemeinen Formel I

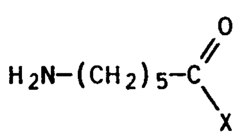 I,

in der X eine OH-Gruppe, die Gruppe $OR^1$, in der $R^1$ für einen Alkylrest, Cycloalkylrest oder Aralkylrest steht oder die Gruppe

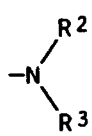 ,

in der $R^2$ und $R^3$ gleich oder verschieden sein können und jeweils für ein Wasserstoffatom oder einen Alkylrest mit 1 bis 4 Kohlenstoffatomen stehen und $R^2$ und $R^3$ zusammen mit dem Stickstoffatom, das sie substituieren, einen 5- bis 7-gliedrigen Ring bilden können oder Gemischen derselben in Gegenwart eines unter Reaktionsbedingungen flüssigen, inerten Reaktionsmediums mit einem Siedepunkt über dem des Caprolactams, wobei man als Reaktionsmedium Kohlenwasserstoffe mit einem Siedepunkt von mindestens 300° C verwendet, eine Temperatur von 150 bis 350° C einhält, 6-Aminocapronsäure, deren Ester oder Amide der Formel I sowie Gemische derselben in dem Maße zuführt, wie sie umgesetzt werden und Caprolactam in dem Maße, wie es entsteht, aus dem Reaktionsgemisch abtrennt.

Das neue Verfahren hat den Vorteil, daß es bei Normaldruck mit hohem Umsatz und hohen Ausbeuten

verläuft, keine aufwendigen Lösungsmittel mitverwendet werden müssen und somit die Wiederaufarbeitung der Lösungsmittel entfällt. Darüber hinaus hat das neue Verfahren den Vorteil, daß sich die Gewinnung von Caprolactam einfach gestaltet.

Das neue Verfahren ist insofern bemerkenswert als in der DE-OS 32 35 938, Seite 18 und 19, ausdrücklich darauf hingewiesen wird, daß bei der Umsetzung von Aminocapronsäureestern in Kohlenwasserstoffen als Lösungsmittel ein unlösliches Polymerisat gebildet wird.

Ausgangsstoffe sind solche der allgmeinen Formel I

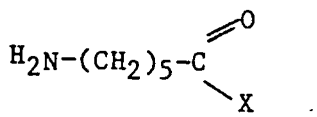

I,

in der X eine OH-Gruppe, die Gruppe $OR^1$, in der $R^1$ für einen Alkylrest, Cycloalkylrest oder Aralkylrest steht oder die Gruppe

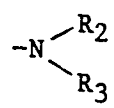

,

in der $R_2$ und $R^3$ gleich oder verschieden sein können und jeweils für ein Wasserstoffatom oder einen Alkylrest mit 1 bis 4 Kohlenstoffatomen stehen und $R^2$ und $R^2$ zusammen mit dem Stickstoffatom, das sie substituieren, einen 5- bis 7-gliedrigen Ring bilden können.

Geeignete Ausgangsstoffe sind beispielsweise 6-Aminocapronsäure, Ester der 6-Aminocapronsäure mit $C_1$- bis $C_{10}$-Alkanolen, Cycloalkanolen mit 3 bis 10 Kohlenstoffatomen oder Aralkanolen mit 7 bis 10 Kohlenstoffatomen. Bevorzugt sind unter den Estern 6-Aminocapronsäure $C_1$- bis $C_4$-Alkylester. Ferner sind geeignet 6-Aminocapronsäureamid sowie mit $C_1$- bis $C_4$-Alkylgruppen substituierte Amide.

Bevorzugte 6-Aminocapronsäureester sind solche mit Ethanol, n-Propanol, i-Propanol, n-Butanol oder sec. Butanol als Esterkomponente. Besonders bevorzugt sind 6-Aminocapronsäuremethyl und -ethylester.

Bevorzugt ist auch 6-Aminocapronsäureamid sowie 6-Aminocapronsäuredimethyl- oder -diethylamid.

Die verwendeten Ausgangsstoffe können sowohl in reiner Form als auch in Form einer wäßrigen oder alkoholischen Lösung eingesetzt werden. Falls man Ester verwendet, entspricht der als Lösungsmittel verwendete Alkohol zweckmäßig der Alkoholkomponente des Esters.

Die Umsetzung wird bei einer Temperatur von 150 bis 350 ° C, vorteilhaft 250 bis 330 ° C durchgeführt. Im allgemeinen hält man bei der Umsetzung Atmosphärendruck ein, doch ist es auch möglich, die Umsetzung unter vermindertem Druck, z.B. bis zu 10 mbar durchzuführen.

Erfindungsgemäß wird die Umsetzung in unter Reaktionsbedingungen inerten flüssigen Kohlenwasserstoffen als Reaktionsmedium durchgeführt. Die verwendeten Kohlenwasserstoffe haben einen höheren Siedepunkt als das herzustellende Caprolactam. Man verwendet Kohlenwasserstoffe mit einem Siedepunkt von mindestens 300 ° C, insbesondere von 350 bis 550 ° C. Geeignete Kohlenwasserstoffe sind beispielsweise Mineralölfraktionen mit dem entsprechenden Siedepunkt, insbesondere technisches Weißöl, Vakuumgasöl, Vakuumrückstandsöl, Heizöl S, geschmolzenes Paraffinwachs, aromatisches Kohlenwasserstofföl oder Marlothermöl.

Vorteilhaft wird die Umsetzung unter Mitverwendung von sauren Katalysatoren durchgeführt. Geeignete Katalysatoren sind beispielsweise Phosphorsäure, Diphenylphosphinsäure, Dodecylbenzolsulfonsäure oder saure Katalysatoren auf Trägern wie z.B. Phosphorsäure auf Silikagel. Die Katalysatoren werden vorteilhaft dem Reaktionsmedium in einer Menge von 0,1 bis 10 Gew.-% zugesetzt.

Erfindungsgemäß werden die Ausgangsstoffe dem auf Reaktionstemperatur erhitzten Kohlenwasserstoff in dem Maße zugeführt, wie sie umgesetzt werden. Vorteilhaft führt man je Liter Kohlenwasserstoff 0,01 bis 1,0 kg/h 6-Aminocapronsäure, deren Ester oder Amide oder Gemische derselben zu. Vorteilhaft führt man zusätzlich Inertgase wie Stickstoff oder Kohlendioxid, insbesondere Stickstoff zu. Es hat sich bewährt, je Liter Kohlenwasserstoff 0,01 bis 0,2 $Nm^3$ Inertgas anzuwenden.

Unmittelbar mit der Umsetzung ist eine Stofftrennung verbunden. Hierdurch wird Caprolactam in dem Maße wie es entsteht aus dem Reaktionsgemisch abgetrennt, z.B. abdestillert oder mit dem Inertgas gestrippt. Zugleich werden andere leichtsiedende Verbindungen z.B. freigesetztes Wasser, freigesetzte Alkohole oder freigesetzte Amine, nichtumgesetzte Ausgangsstoffe sowie geringe Mengen leichtflüchtiger

Bestandteile aus dem Lösungsmittel gasförmig entfernt. Auf diese Weise wird die Konzentration an 6-Aminocapronsäure, deren Estern oder Amiden sowie Caprolactam im erhitzten Kohlenwasserstoff möglichst klein gehalten. Die entstehenden Brüden, die neben dem Inertgas Caprolactam und die vorgenannten Stoffe enthalten, werden anschließend kondensiert und vorteilhaft einer Destillationskolonne zugeführt und Caprolactam abgetrennt.

Die Umsetzung führt man beispielsweise in Rührbehältern oder zylindrischen Reaktoren oder Füllkörperkolonnen durch. Diese sind zweckmäßig bis zu 2/3 mit dem als Reaktionsmedium verwendeten inerten Kohlenwasserstoff, der gegebenenfalls Katalysatoren enthält, beschickt. Die Ausgangsprodukte werden vorteilhaft von unten, gegebenenfalls mit einem Inertgas wie Stickstoff als Strippgas dem Reaktor zugeführt. Das flüssige Reaktionsgemisch wird auf der vorgenannten Temperatur gehalten und die ausgetragenen Produkte nach Kondensation durch Destillation getrennt. Geringe Mengen schwerflüchtiger Nebenprodukte bleiben in dem Kohlenwasserstoff zurück und werden vorteilhaft durch partielles Abziehen des Kohlenwasserstoffs ausgeschleust und der Reaktorinhalt mit frischem Kohlenwasserstoff ergänzt. Der abgezogene Kohlenwasserstoff wird nicht zurückgewonnen sondern zweckmäßig für die Unterfeuerung verwendet.

Caprolactam wird zur Herstellung von Polycaprolactam verwendet.

Das Verfahren nach der Erfindung sei an folgenden Beispielen veranschaulicht.

Beispiel 1

Ein Rührkolben von 2 l Inhalt wurde mit 900 g technischem Weißöl beschickt und auf 250 °C erhitzt. Stündlich wurden 73 g 6-Aminocapronsäuremethylester zusammen mit 110 Nl Stickstoff von unten zugeführt. Die das Reaktionsgefäß verlassenden gasförmigen Produkte wurden abgekühlt und das Kondensat gaschromatographisch untersucht. Nach einer Versuchsdauer von 4 Stunden erhielt man 295 g Kondensat, das laut gaschromatographischer Analyse 6,4 g nicht umgesetzten 6-Aminocapronsäuremethylester und 220,6 g Caprolactam enthielt. Dies entspricht einem Umsatz von 97,8 % und einer Selektivität von 99,1 %.

Beispiel 2

Ein Rührkolben von 2 l Inhalt wurde mit 900 g technischem Weißöl beschickt und auf 250 °C erhitzt. Stündlich wurden 76 g einer 61 %igen methanolischen Lösung von 6-Aminocapronsäuremethylester zusammen mit 50 Nl Stickstoff von unten zugeführt. Die das Reaktionsgefäß verlassenden gasförmigen Produkte wurden kondensiert. In 4 Stunden wurden insgesamt 304 g der Lösung, entsprechend 185,4 g 6-Aminocapronsäuremethylester, umgesetzt. Man erhielt 292 g Kondensat, das laut gaschromatographischer Analyse 10,7 g nicht umgesetzten 6-Aminocapronsäuremethylester und 132,7 g Caprolactam enthielt. Dies entspricht einem Umsatz von 94,2 % und einer Selektivität von 97,5%.

Beispiel 3

Ein Rührkolben von 2 l Inhalt wurde mit 900 g Vakuumgasöl beschickt und auf 300 °C beheizt. Stündlich wurden 49 g einer 67 %igen methanolischen Lösung von 6-Aminocapronsäuremethylester zusammen mit 50 Nl Stickstoff von unten zugeführt. Die das Reaktionsgefäß verlassenden gasförmigen Produkte wurden kondensiert. In 3 Stunden wurden insgesamt 147 g der Lösung, entsprechend 98,5 g 6-Aminocapronsäuremethylester, umgesetzt. Man erhielt 154 g Kondensat, das laut gaschromatographischer Analyse 5,8 g nicht umgesetzten 6-Aminocapronsäuremethylester und 70,5 g Caprolactam enthielt. Dies entspricht einem Umsatz von 94,1 % und einer Selektivität von 97,6 %.

Beispiel 4

Ein Rührkolben von 2 l Inhalt wurde mit 900 g technischem Weißöl und 10 g Diphenylphosphinsäure beschickt und auf 300 °C beheizt. Stündlich wurden 87 g einer 57%igen methanolischen Lösung von 6-Aminocapronsäuremethylester zusammen mit 50 Nl Stickstoff von unten zugeführt. Die das Reaktionsgefäß verlassenden gasförmigen Produkte wurden kondensiert. In 3 Stunden wurden insgesamt 261 g der Lösung, entsprechend 148,8 g 6-Aminocapronsäuremethylester, umgesetzt. Man erhielt 262 g Kondensat, das laut gaschromatographischer Analyse 0,7 g nicht umgesetzten 6-Aminocapronsäuremethylester und 106,1 g Caprolactam enthielt. Dies entspricht einem Umsatz von 99,5 % und einer Selektivität von 91,9%.

Beispiel 5

Ein Rührkolben von 2 l Inhalt wurde mit 900 g technischem Weißöl beschickt und auf 250 °C erhitzt. Stündlich wurden 80 g 6-Aminocapronsäureethylester zusammen mit 110 Nl Stickstoff von unten zugeführt. Die das Reaktionsgefäß verlassenden gasförmigen Produkte wurden kondensiert. Nach einer Versuchsdauer von 4 Stunden erhielt man 325 g Kondensat, das laut gaschromatographischer Analyse 8,0 g nicht umgesetzten 6-Aminocapronsäureethylester und 219,4 g Caprolactam enthielt. Dies entspricht einem Umsatz von 97,5 % und einer Selektivität von 98,9 %.

Beispiel 6

Ein Rührkolben von 2 l Inhalt wurde mit 900 g technischem Weißöl beschickt und auf 250 °C erhitzt. Stündlich wurden 100 g einer 30 %igen wäßrigen Lösung von 6-Aminocapronsäure von unten zugeführt. Die das Reaktionsgefäß verlassenden gasförmigen Produkte wurden kondensiert. In 3 Stunden wurden insgesamt 300 g der Lösung, entsprechend 90 g 6-Aminocapronsäure, umgesetzt. Man erhielt 280 g Kondensat, das laut analytischer Gaschromatographie 56,8 g Caprolactam aber kein Ausgangsprodukt enthielt. Dies entspricht bei quantitativem Umsatz einer Selektivität von 73,1 %.

Beispiel 7

Ein Rührkolben von 2 l Inhalt wurde mit 900 g technischem Weißöl beschickt und auf 300 °C erhitzt. Stündlich wurden 65 g 6-Aminocapronsäure zusammen mit 110 Nl Stickstoff zugeführt. Die das Reaktionsgefäß verlassenden gasförmigen Produkte wurden kondensiert. Nach einer Versuchsdauer von 4 Stunden erhielt man 231 g Kondensat, das laut analytischer Gaschromatographie 190,9 g Caprolactam aber kein Ausgangsprodukt enthielt. Dies entspricht bei quantitativem Umsatz einer Selektivität von 85,1 %.

Beispiel 8

Ein Rührkolben von 2 l Inhalt wurde mit 900 g technischem Weißöl beschickt und auf 300 °C erhitzt. Stündlich wurden 65 g 6-Aminocapronsäureamid zusammen mit 110 Nl Stickstoff zugeführt. Die das Reaktionsgefäß verlassenden gasförmigen Produkte wurden abgekühlt und das Kondensat gaschromatographisch untersucht. Nach einer Versuchsdauer von 4 Stunden erhielt man 225 g Kondensat, das laut gaschromatographischer Analyse 215,8 g Caprolactam aber kein Ausgangsprodukt enthielt. Dies entspricht bei quantitativem Umsatz einer Selektivität von 95,5 %.

Beispiel 9

Ein beheizbares Rohr mit einem inneren Durchmesser von 60 mm und einer Länge von 1 500 mm wurde mit 2 000 g technischem Weißöl beschickt und auf 300 °C beheizt. Stündlich wurden 145 g 6-Aminocapronsäuremethylester zusammen mit 110 Nl Stickstoff von unten zugeführt. Am Kopf des Rohres wurden die gasförmigen Produkte abgezogen, abgekühlt und das Kondensat gaschromatographisch untersucht. Nach einer Versuchsdauer von 4 Stunden erhielt man 595 g Kondensat, das laut gaschromatographischer Analyse 2,1 g nicht umgesetzten 6-Aminocapronsäuremethylester und 446,8 g Caprolactam enthielt. Dies entspricht einem Umsatz von 99.6 % und einer Selektivität von 99,2 %.

**Patentansprüche**

**1.** Verfahren zur Herstellung von Caprolactam durch Erhitzen von 6-Aminocapronsäure, deren Estern oder Amiden der allgemeinen Formel I

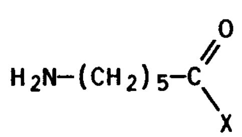

I,

in der X eine OH-Gruppe, die Gruppe $OR^1$, in der $R^1$ für einen Alkylrest, Cycloalkylrest oder Aralkylrest steht oder die Gruppe

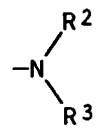 ,

in der $R^2$ und $R^3$ gleich oder verschieden sein können und jeweils für ein Wasserstoffatom oder einen Alkylrest mit 1 bis 4 Kohlenstoffatomen stehen und $R^2$ und $R^3$ zusammen mit dem Stickstoffatom, das sie substituieren, einen 5- bis 7-gliedrigen Ring bilden können oder Gemischen derselben in Gegenwart von einem unter Reaktionsbedingungen flüssigen, inerten Reaktionsmedium mit einem Siedepunkt über dem des Caprolactams, dadurch gekennzeichnet, daß man als Reaktionsmedium Kohlenwasserstoffe mit einem Siedepunkt von mindestens 300 °C verwendet, eine Temperatur von 150 bis 350 °C einhält, 6-Aminocapronsaure, der deren Ester oder Amide der Formel I sowie deren Gemische in dem Maße zuführt, wie sie umgesetzt werden und Caprolactam in dem Maße, wie es entsteht, aus dem Reaktionsgemisch abtrennt.

2. Verfahren nach Anspruch 1, dadurch gekennzeichnet, daß man Kohlenwasserstoffe mit einem Siedepunkt von 350 bis 550 °C verwendet.

3. Verfahren nach den Ansprüchen 1 und 2, dadurch gekennzeichnet, daß man eine Temperatur von 250 bis 330 °C einhält.

4. Verfahren nach den Ansprüchen 1 bis 3, dadurch gekennzeichnet, daß man je Liter Kohlenwasserstoffe stündlich 0,01 bis 1,0 kg Aminocapronsäure, deren Ester oder Amide oder Gemische derselben zuführt.

5. Verfahren nach den Ansprüchen 1 bis 4, dadurch gekennzeichnet, daß man zusätzlich Inertgase zuführt.

6. Verfahren nach den Ansprüchen 1 bis 5, dadurch gekennzeichnet, daß man verminderten Druck anwendet.

7. Verfahren nach den Ansprüchen 1 bis 6, dadurch gekennzeichnet, daß man zusätzlich saure Katalysatoren mitverwendet.

8. Verfahren nach den Ansprüchen 1 bis 7, dadurch gekennzeichnet, daß man 6-Aminocapronsäuremethyl- oder -ethylester als Ausgangsverbindungen verwendet.

9. Verfahren nach den Ansprüchen 1 bis 7, dadurch gekennzeichnet, daß man 6-Aminocapronsäureamid, das am Stickstoffatom durch Methyl- oder Ethylgruppen substituiert sein kann, als Ausgangsstoff verwendet.

10. Verfahren nach den Ansprüchen 1 bis 9, dadurch gkennzeichnet, daß man einen Teil des schwerflüchtige Nebenprodukte enthaltenden Kohlenwasserstoffs ausschleust und durch frischen Kohlenwasserstoff ersetzt, und den ausgeschleusten Kohlenwasserstoff unterfeuert.

## Claims

1. A process for preparing caprolactam by heating 6-aminocaproic acid, ester or amide of the formula I

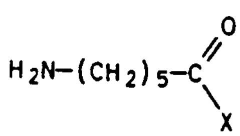 (I)

where X is OH, $OR^1$, where $R^1$ is alkyl, cycloalkyl or aralkyl, or

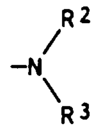
,

where $R^2$ and $R^3$, identical or different, are singly hydrogen or alkyl of 1 to 4 carbon atoms or together, combined with the nitrogen atom on which they are substituents, a 5-, 6- or 7-membered ring, or a mixture thereof, in the presence of an inert reaction medium which is liquid under the reaction conditions and has a boiling point above that of caprolactam, which comprises using as the reaction medium a hydrocarbon having a boiling point of not less than 300 ° C, maintaining a temperature of from 150 to 350 ° C, charging the 6-aminocaproic acid, ester or amide of the formula I or the mixture thereof at a rate commensurate with the rate of conversion, and separating caprolactam from the reaction mixture at a rate commensurate with its rate of formation.

**2.** A process as claimed in claim 1, wherein the hydrocarbon used has a boiling point from 350 to 550 ° C.

**3.** A process as claimed in claim 1 or 2, wherein a temperature from 250 to 330 ° C is maintained.

**4.** A process as claimed in claim 1 or 2 or 3, wherein per hour from 0.01 to 1.0 kg of aminocaproic acid, ester, amide or mixture thereof is introduced into the hydrocarbon per liter thereof.

**5.** A process as claimed in claim 1 or 2 or 3 or 4, wherein an inert gas is introduced in addition.

**6.** A process as claimed in claim 1 or 2 or 3 or 4 or 5, wherein reduced pressure is employed.

**7.** A process as claimed in claim 1 or 2 or 3 or 4 or 5 or 6, wherein an acid catalyst is additionally present.

**8.** A process as claimed in claim 1 or 2 or 3 or 4 or 5 or 6 or 7, wherein the starting compound is methyl 6-aminocaproate or ethyl 6-aminocaproate.

**9.** A process as claimed in claim 1 or 2 or 3 or 4 or 5 or 6 or 7, wherein the starting material used is 6-aminocaproamide which may be substituted on the nitrogen atom by methyl or ethyl.

**10.** A process as claimed in claim 1 or 2 or 3 or 4 or 5 or 6 or 7 or 8 or 9, wherein a portion of the hydrocarbon containing sparingly volatile by-products is bled out and replaced by fresh hydrocarbon and the hydrocarbon bleedout is used for undergrate firing.

## Revendications

**1.** Procédé de préparation de caprolactame par chauffage d'acide 6-aminocaproïque, d'esters ou d'amides de celui-ci de formule générale I

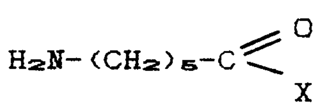
I

dans laquelle X représente un groupement OH, le groupement $OR^1$ - où $R^1$ est mis pour un reste alkyle, pour un reste cycloalkyle ou pour un reste aralkyle - ou le groupement

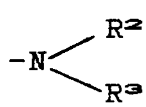
,

où $R^2$ et $R^3$ peuvent être identiques ou différents et sont mis chacun pour un atome d'hydrogène ou un reste alkyle ou peuvent former ensemble, avec l'atome d'azote qu'ils substituent, un noyau penta- à

heptagonal, ou de mélanges de ces composés, en présence d'un milieu réactionnel inerte, liquide dans les conditions de la réaction et ayant un point d'ébullition supérieur à celui du caprolactame, caractérisé en ce qu'on utilise, conme milieu réactionnel, des hydrocarbures ayant un point d'ébullition d'au moins 300°C, on maintient une température de 150 à 350°C, on introduit l'acide 6-aminocaproïque, ses esters ou ses amides de formule I dans la mesure où ils sont transformés et on sépare du mélange réactionnel le caprolactame dans la mesure où il est formé.

**2.** Procédé selon la revendication 1, caractérisé en ce qu'on utilise des hydrocarbures ayant un point d'ébullition de 350 à 550°C.

**3.** Procédé selon l'une ou l'autre des revendications 1 et 2, caractérisé en ce qu'on maintient une température de 250 à 330°C.

**4.** Procédé selon l'une quelconque des revendications 1 à 3, caractérisé en ce qu'on introduit par litre d'hydrocarbure et par heure de 0,01 à 1,0 kg d'acide aminocaproïque, de ses esters ou amides ou de leurs mélanges.

**5.** Procédé selon l'une quelconque des revendications 1 à 4, caractérisé en ce qu'on introduit en outre des gaz inertes.

**6.** Procédé selon l'une quelconque des revendications 1 à 5, caractérisé en ce qu'on utilise une pression réduite.

**7.** Procédé selon l'une quelconque des revendications 1 à 6, caractérisé en ce qu'on utilise également et simultanément des catalyseurs acides.

**8.** Procédé selon l'une quelconque des revendications 1 à 7, caractérisé en ce qu'on utilise comme substance de départ du 6-aminocaproate de méthyle ou d'éthyle.

**9.** Procédé selon l'une quelconque des revendications 1 à 7, caractérisé en ce qu'on utilise comme substance de départ du 6-aminocapramide qui peut être substitué sur l'atome d'azote par des groupements méthyle ou éthyle.

**10.** Procédé selon l'une quelconque des revendications 1 à 9, caractérisé en ce qu'on enlève une partie de l'hydrocarbure contenant des produits secondaires peu volatils et on remplace par de l'hydrocarbure frais, et on utilise l'hydrocarbure séparé comme combustible au foyer inférieur.